(19) Europäisches Patentamt

European Patent Office

Office européen des brevets

(11) **EP 1 615 061 A1**

(12) **EUROPEAN PATENT APPLICATION**
published in accordance with Art. 158(3) EPC

(43) Date of publication:
**11.01.2006 Bulletin 2006/02**

(51) Int Cl.:
*G02B 21/00* (1968.09)    *C12M 1/34* (1980.01)

(21) Application number: **04723379.6**

(22) Date of filing: **25.03.2004**

(86) International application number:
**PCT/JP2004/004209**

(87) International publication number:
**WO 2004/086116 (07.10.2004 Gazette 2004/41)**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **27.03.2003 JP 2003088929**
**18.08.2003 JP 2003207738**

(71) Applicant: **Effector Cell Institute**
**Tokyo 153-0041 (JP)**

(72) Inventors:
• **KANEGASAKI, Shiro**
  **1530041 (JP)**
• **HAYASHI, Shinichi;**
  **uya-ku, Tokyo;**
  **1510072 (JP)**

(74) Representative: **Hackney, Nigel John et al**
**Mewburn Ellis LLP**
**York House,**
**23 Kingsway**
**London WC2B 6HP (GB)**

(54) **OBSERVING TOOL AND OBSERVING METHOD USING SAME**

(57) A transparent small object such as a cell can be simply observed without using any special modulating element needed for special observing methods. An observing tool for containing an object to be observed is used for a method for observing the object by illuminating the object with vertical illuminating light through an optical system having an objective lens. The observing tool has a reflective surface that reflects vertical illumination light when the object is observed. The reflective surface is provided on the front surface of the observing tool facing to the objective lens or on the back opposed to the front surface. The observing tool has a container holding a liquid. Using the observing tool, a cell or the like can be observed along with the culture solution.

**FIG. 1**

EP 1 615 061 A1

## Description

### Technical Field

The present invention relates to an observation technique of a material body, and in particular, it relates to an observation technique of a transparent micro-object such as a cell.

### Background

Conventionally, a transmission observation microscope has been used for observing a micro transparent object such as a cell. In order to observe an internal structure or a form of living unstained cell with high contrast, it has been necessary to use a microscope provided with a particular kind of lens, together with a particular kind of condenser having a phase contrast ring and/or a differential interference prism, such as a phase-contrast microscope, relief phase contrast microscope, differential interference microscope, and polarization microscope.

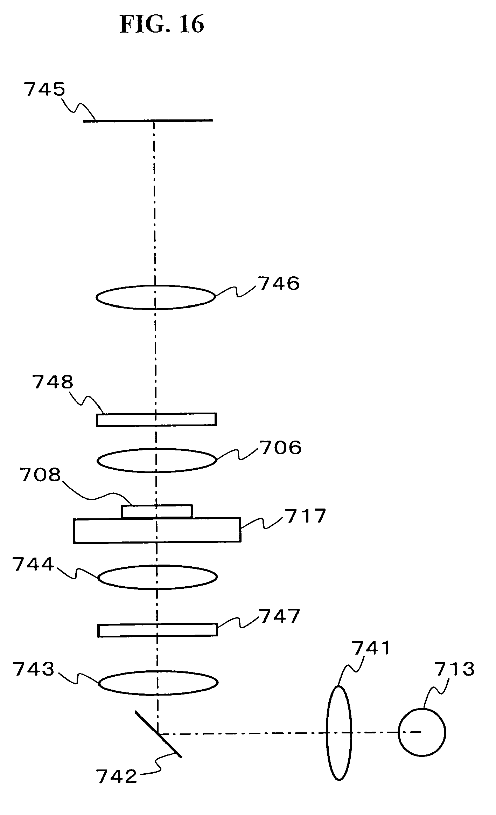

Specifically, a special observing method is widely employed, for example, represented by a phase contrast method, focal illumination method, and differential interference method, as a method which observes by use of a microscope, a transparent micro-object such as a cell in a culture solution (see Japanese Patent Laid-open Publication No. H07-225341). As shown in FIG. 16, an optical system of the special observing method has a configuration for general transmission bright field observation, including a light source 713 which generates illumination lights, a collimating lens 741 which allows the illumination lights generated by the light source 713 to proceed to the same traveling direction, a reflecting mirror 742 which deflects the traveling direction of the illumination lights proceeding to the same direction into the perpendicular direction, a window lens 743 which collects the illumination lights, a condenser lens 744 which irradiates a sample 708 including an object to be observed with the illumination lights thus collected, an objective lens 706 which extends and projects the sample 708, an imaging lens 746 which forms an image of the sample 708 on a field 745, and a stage 717 which adjusts a position for observing the sample 708, and this configuration is added with an illumination modulation element 747 at an entrance pupil location of the condenser lens 744, and an image formation modulation element 748 at an exit pupil location of the objective lens 706. The phase contrast method uses a ring slit as the illumination modulation element 747 and a phase plate as the image formation modulation element 748. The focal illumination method uses a decentering opening as the illumination modulation element 747. The differential interference method uses a polarizing plate and a differential interference prism for both of the illumination modulation element 747 and the image formation illumination element 748. By use of the special observing methods as described above, it is possible to observe even a transparent body by enhancing contrast, as far as the target body has a refractive index which is different from a surrounding material. These special observing methods are effective to observe with enhanced contrast, a biomedical tissue that is transparent under normal conditions and hardly visible as it is.

### Summary of the Invention

However, in these special observing methods, particular modulation elements should be disposed at the lens pupil of the condenser lens 744 and that of the objective lens 706 as described above. These particular modulation elements are hugely expensive, and in many cases, they are also inconvenient in usage, such that the modulation elements should be switched, for example, every time when a magnification of the objective lens 706 is changed.

The present invention has been made considering the situation above, and an object of the present invention is to provide a technique which allows an observation of a transparent micro-object without using the particular modulation elements required for the special observing method, and achieves a simple observation.

The observing tool according to the present invention is provided with an observation target storage section having a mirror (reflection plane). The observing tool according to the present invention is used for storing the observation target that is employed in an observing method in which the observation target is observed while being irradiated with vertical lighting via an optical system having an objective lens, and the observing tool is provided with a reflection plane to reflect the vertical lighting when the observation is performed.

The reflection plane may be provided on a surface that is to be facing to the objective lens when the observation is performed. Alternatively, it may be provided on the surface opposite to the surface that is facing to the objective lens.

In addition, a flow channel may be formed for the observation target to pass through. The storage section for storing the observation target may be provided with an inlet through which liquid containing the observation target is injected and an outlet through which the liquid is run off.

The observing method according to the present invention is characterized in that an observation target is observed being illuminated with a vertical lighting via an optical system having an objective lens, and the observing tool to store the observation target is provided with a reflection plane to reflect the vertical lighting when the observation is performed, and the observation target is stored in the observing tool and this observation target is observed.

The reflection plane may be provided on a surface which is to be facing to the objective lens when the observation is performed, or may be provided on a surface opposite to the surface that is to be facing to the objective lens.

Furthermore, the objective target may be a micro transparent object.

In addition, the observing tool has a container to hold liquid, and the liquid including the observation target may be stored in this container. Here, the observation target may be a cell and the liquid may be a culture solution.

The observation target may be stored in the observing tool so that a distance between the observation target and the reflection plane becomes a half or less than the focal depth of the optical system.

Specifically, the observation target may be stored in the observing tool so that distance d between the observation target and the reflection plane satisfies the following formula (1).

$$d \leq W/(2NA^2) \ \ldots \ (1)$$

(In the formula, d represents a distance between the observation target and the reflection plane, $W$ represents a wavelength of the light employed in the observation, and $NA$ represents a numerical aperture of the optical system.)

It is further possible to store the observation target in the observing tool so that the numerical aperture of the illumination light against the observation target becomes smaller than the numerical aperture of the objective lens.

Specifically, the observation target is stored in the observing tool so that the distance d between the observation target and the reflection plane satisfies the following formula (2).

$$d > F/(4\tan(\sin^{-1} NA)) \ \ldots \ (2)$$

(In the formula, d represents a distance between the observation target and the reflection plane, $F$ represents a visual field diameter of the optical system, and $NA$ represents the numerical aperture of the optical system.)

According to the present invention, a transparent micro-object such as a cell can be observed with an enhanced contrast, even if a special optical means is not employed.

In other words, when the cell observing tool according to the present invention is employed, by way of a general optical microscope or a monitor using a general lens and CCD camera, a cell can be observed with a clear picture even containing a granule component, the cell including, for example, a blood cell such as heterophilic leucocyte, acidophilic leucocyte, basophil, monocyte, macrophage, lymphocytea, and other animal cell, or a protoplast of a plant or the like. Therefore, it is not necessary to use a special device such as phase contrast microscope as conventionally used.

**Brief Description of the Drawings**

FIG.1 (A) to FIG. (C) are cross sectional views of observing tool according to the first embodiment.

FIG. 2(A) and (B) are cross sectional views of the observing tool having a structure 40 with a mirror plane.

FIG. 3 (A1) to (C) are cross sectional views of the observing tool having a cover for covering the storage section.

FIG. 4 is another usage example of the observing tool according to the first embodiment, and (A) is a cross sectional view showing that the structure 1 is placed on a cover glass.

(B) is a cross sectional view of conceptual diagram showing the situation where a hole allowing liquid to pass through is provided. (C) is a perspective view of the observing tool as shown in (B).

FIG. 5 is a top view of one example of the observing tool which has a storage section marked with a scale.

FIG. 6(A) is a cross sectional view of the observing tool which is formed in a shape of tube as a whole.

(B) is a cross sectional view taken along dotted line AB as shown in (A).

FIG. 7 is a diagram showing a configuration of an observing apparatus according to the second embodiment.

FIG. 8 is a diagram showing a configuration of optical system of the observing method according to the present invention.

FIG. 9 is an illustration showing a culture plate and cultured cell.

FIG. 10 is a diagram showing how to form contrast to an object, according to the present invention.

FIG. 11(a) is a diagram showing a calculation model of simulation for image formation in the second embodiment.

FIG. 11(b) and (c) are diagrams showing calculation results of the simulation for image formation.

FIG. 12(a) is a diagram showing a calculation model of the simulation for image formation in the second embodiment.

FIG. 12 (b) to (f) are diagrams showing calculation results of the simulations for image formation, when the distance between the cell 302 and the mirror coating 307 are 1.0$\mu$m, 1.2$\mu$m, 1.4$\mu$m, 1.6$\mu$m and 1.8$\mu$m, respectively.

FIG. 13 is a diagram showing a decrease of the numerical aperture of the illumination light, which is decreased by the reflection plane.

FIG.14 (a) is an illustration showing a calculation model used for a simulation example of an object image, when the numerical aperture of the illumination light is smaller than the numerical aperture of the objective lens. FIG. 14 (b) to (f) are diagrams showing calculation results of simulations of image formation, respectively when the numerical aperture of the illumination light is 100%, 80%, 60%, 40%, and 20% of the numerical aperture of the objective lens.

FIG. 15 is an illustration showing a configuration of the micro flow-channel observing apparatus according to the third embodiment.

FIG. 16 is a diagram showing a configuration of optical system of a conventional special observing method.

FIG. 17 is a diagram showing how to form a contrast to an object, according to a conventional transmission observation microscope.

FIG. 18 is a photograph taken when eosinophilic cells are observed, by use of the observing tool as shown in FIG. 3 (A2).

**Preferred Embodiments of the Invention**

One embodiment of the present invention will be explained, with reference to the accompanying drawings. Firstly, an observing tool relating to the first embodiment will be explained, to which the present invention has been applied.

The observing tool according to the present invention is to observe and detect a cell and the like with a reflected light, by use of a microscope, and it is provided with a part for storing the cell and the like, that is, an observation target storage section. It is at least provided with a plane, that is, a mirror plane (reflection plane) to reflect a light within a wavelength area to be observed by the observing tool.

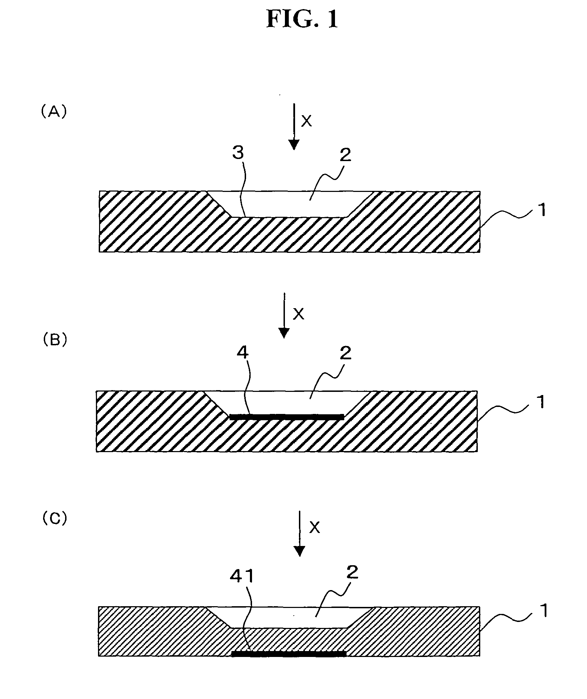

One configuration example of the observing tool relating to the present invention is shown in FIG. 1 to FIG. 6. In the figures, the arrow X indicates that the observation target is observed from the X direction by use of the objective lens.

In the observing tool as shown in FIG. 1(A), reference numeral 1 indicates a structure made of glass, plastic, metal, silicon wafer, or the like, and reference numeral 2 indicates a depressed area to store an observation target such as a cell. A mirror to reflect a light within a wavelength area to be observed is installed on the bottom surface 3 of the storage section 2. The mirror to provide the reflection plane is, as a way of example, a glass, plastic, metal, or the like which is coated with metal plating such as silver plating and chrome plating, or to which a metal foil is adhered, so as to form a mirror plane. As an alternative example, the structure 1 may be made of a material such as metal and silicon wafer which itself is available for specular working, and when it is processed to provide a depressed area, the bottom surface as it is forms a mirror, or after the depressed area is formed, the bottom surface of the depressed area is subjected to the specular working. Here, it is sufficient that at least the bottom surface 3 is a mirror, and thus the entire surface of the structure may be a mirror.

When the structure 1 of FIG. 1(A) is made of a silicon wafer, it is prepared by processing the silicon wafer, for example, by a conventional method such as machine polishing and chemistry etching, so as to form a depressed area for storing a cell. For example, in the case of FIG. 1(A), the bottom surface of the silicon wafer is in a specular state, thereby forming the mirror 3. If the bottom surface is not in a specular state sufficiently even after the process for forming the depressed area as described above, further process may be given as appropriate, so that the bottom surface of the depressed area is provided with a mirror finish.

The size of the structure is sufficient, if it is available for a general microscope. Preferably, the storage section may have a depth, for example, to allow cells to be arranged in one layer, in order to observe a cellular granular structure in detail. For example, the storage section has a depth of 1 to 100$\mu$m, and if it is circular in shape, the diameter of 1 to 5mm is sufficient. However, this size is not limited to those above and an appropriate size is selectable as required.

A material for the structure 1 may be sufficient if it is processible for specular (reflection plane) working, or it is a material on which a reflection plane can be formed by plating and the like. For example, an inorganic compound such as glass and quartz; a plastic such as polystyrene, methacryl resin, polypropylene, polyethylene, vinyl chloride resin, polyphenylene ether, and polyphenylene sulfide; a metal or alloy such as stainless steel, aluminum, bronze; and a nonmetal inorganic material such as ceramics can be used.

In the observing tool as shown in FIG. 1(B), the structure 1 is made of a glass, plastic, and the like, for example, and the storage section 2 is provided. Furthermore, a reflecting layer made of a material having a flat and smooth surface with a high reflectance is formed on the bottom 4 of the storage section 2. For example, a foil or film of silicon wafer is adhesively bonded or silver plating and the like are applied.

The observing tool as shown in FIG. 1 (C) is characterized in that a reflection plane is provided on a surface 41 opposite to the surface which is to be facing to the objective lens. The structure 1 is made of a material which allows a light within the wavelength area to be observed to pass through. For example, it is made of an inorganic compound such as glass and quartz, and a plastic such as polystyrene, methacrylic resin, polypropylene, polyethylene, chloroethylene, polyphenylene ether, and polyphenylene sulfide. With this observing tool, the reflection plane is not scratched even in the case such as cleaning the storage section, and the reflection plane can be maintained easily.

The reflection plane 41 can be formed by plating to be suitable for the material of the structure 1. As for the plating, metal plating is taken as an example, with a material such as silver, which provides a smooth surface and high reflectance.

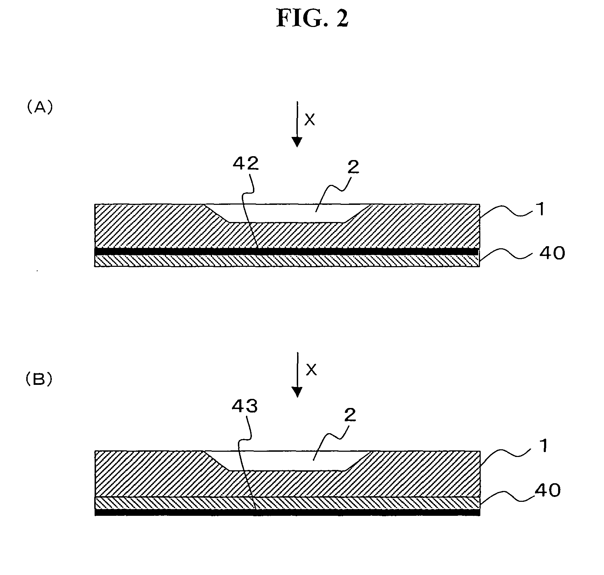

The observing tool as shown in FIG. 2(A) is provided with a reflection plane between the structure 1 which transmits the light, and a structure 40. This observing tool is produced by bonding the structure 40 on which the reflection plane is formed, on the opposite surface of the storage section 2 of the structure 1. Since the structure 1 and the structure 40 are processed separately, and finally they are bonded together, the observing tool can be easy produced. For example, the structure 1 and the structure 40 may be bonded each other via an optics adhesive, such as balsam, photo-curable type synthetic resin adhesive, and the like. Alternatively, the structure 1 and the structure 40 are used in such a manner as superimposing one on another without using the adhesive and the like, when the observation is performed. If they are used by superimposing one on another, the structure 1 and the structure 40 are used while being held and fixed in superimposed manner via a fixture such as a clip.

The observing tool as shown in FIG. 2(B) is similar to the observing tool as shown in FIG. 2 (A), but they are different in a point that the reflection plane is provided on a surface 43 opposite to the surface facing to the objective lens, of the structure 40 which transmits a light. The structure 1 is made of a material which transmits a light. According to this observing tool, a distance between the position of the observation target and the reflection plane can be easily controlled, by adjusting the thickness of the structure 40.

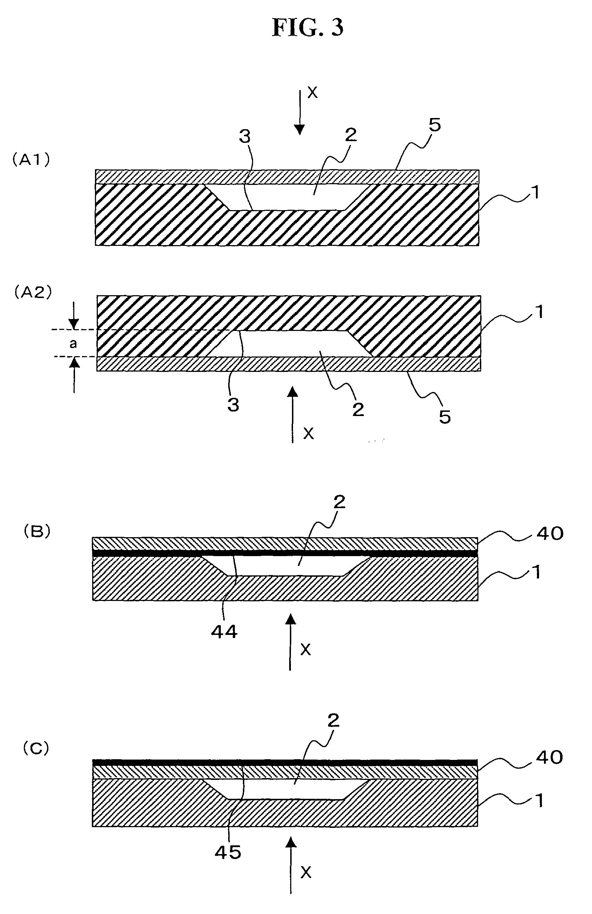

When the observing tool according to the present embodiment is utilized for observing a cell, the cell and a culture solution are together put in the storage section, covers the storage section with a cover glass (numeral 5 in FIG. 3) as shown in FIG. 3 (A1), and a general microscope is used to observe or detect the cell while employing a lighting system which introduces a visible light into the objective lens. Alternatively, the cell is monitored by use of a CCD camera coupled with the objective lens. As the lighting system to introduce the visible light, "Nikon EPI-U" manufactured by Nihon Corporation can be utilized, for instance, being commercially available as a universal lighting system.

FIG. 3(A1) shows a case where the cover glass 5 is installed on the top of the structure 1. However, another usage is possible as shown in FIG. 3 (A2) that the entire apparatus is turned upside down and observation is made from the lower side.

In addition, the observing tool may be the one as shown in FIG. 3 (B) or FIG. 3 (C). The observing tool as shown in FIG. 3(B) is configured such that after the observation target is put into the depressed area of the storage section 2, the structure 40 having the reflection plane on the surface 44 facing to the objective lens covers the storage section 2.

As for the cell observing tool as shown in FIG. 3 (C), it is provided with a mirror plane on the opposite surface 45 of the surface facing to the objective lens, of the structure 40 which transmits a light. According to this observing tool, a distance between the observation target position and the reflection plane can be easily changed by adjusting the thickness of the structure 40.

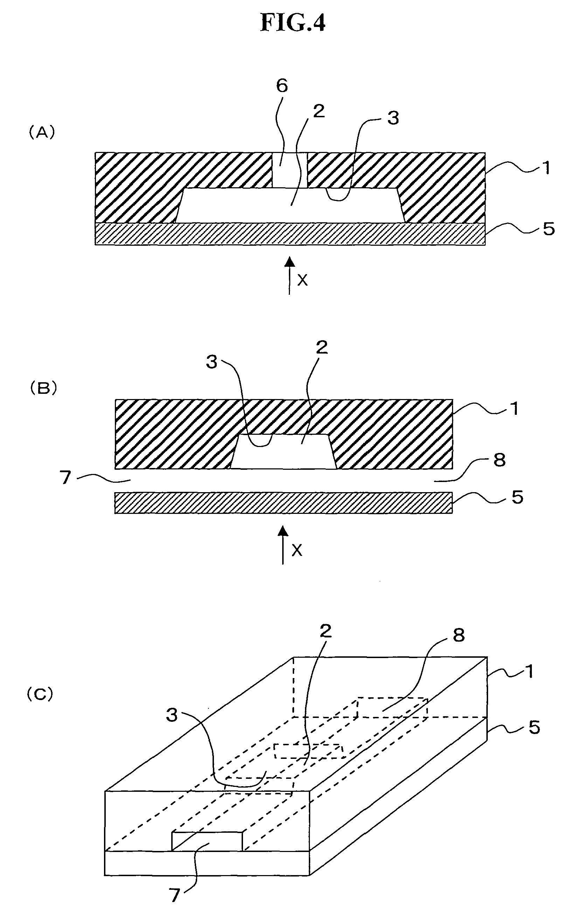

As shown in FIG. 4(A), the observing tool according to the present embodiment can be used, being provided with an inlet 6 to inject the observation target, and placing the structure 1 on the cover glass 5. In the case above, the observation is performed from the downward direction. For example, since the cell can be injected together with the culture solution from the inlet 6, observation operation can be simplified.

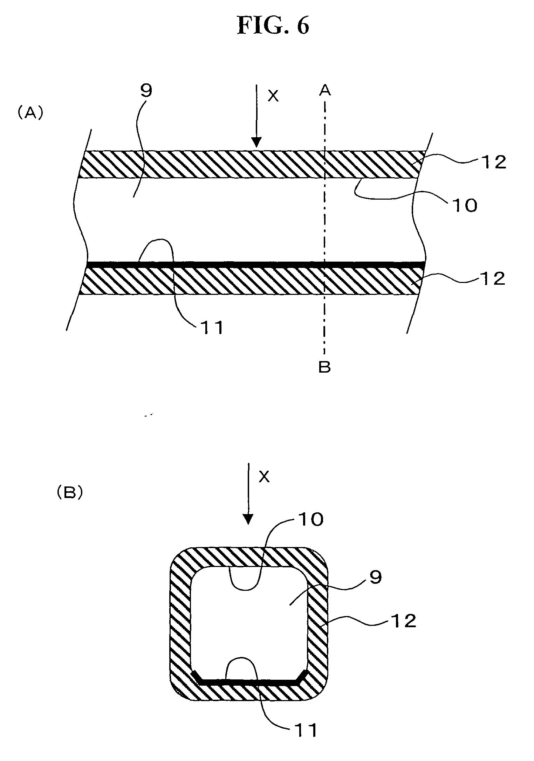

When the observation is performed by use of the observing tool according to the present embodiment, it is not necessary that the observation target, a cell for example, adheres to the glass surface, and it is also possible to observe the cell in a status of floating, that is, in a status of flowing in a liquid, for instance. In other words, as shown in FIG. 4(B) and (C), the structure 1 is provided with holes 7 and 8 to allow the liquid to pass through, allowing the liquid including the cell to flow from one of the holes, and a condition of the cell in the flow can be observed. For this purpose, as shown in FIG. 6, the observing tool may have the reflection plane formed on the inner wall 11 of transparent tube 12 with a cell storage section 9.

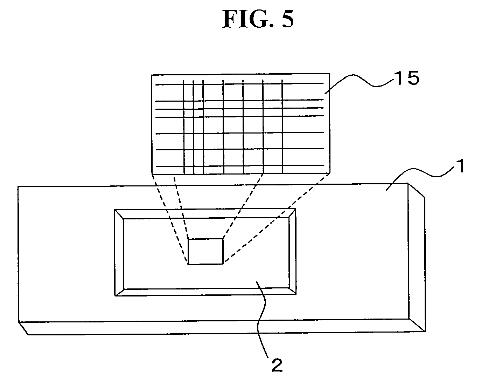

The observing tool as explained above may be configured such that the storage section is marked with a scale as appropriate, as shown in FIG. 5, so that the cells and the like can be estimated. Also in this case, according to the observing tool according to the present embodiment, a micro transparent object can be observed with enhanced contrast, it can be estimated more easily.

When the cell observing tool according to the present invention is employed, in the case where the observation is performed with the naked eye by way of a normal optical microscope and in the case where it is performed by a monitor using a normal lens and CCD camera, a cell can be observed with a clear picture even containing a granule component, the cell including, for example, a blood cell such as heterophilic leucocyte (neutrophil), acidophilic leucocyte (eosinophil), basophil, monocyte, macrophage, lymphocyte, and other animal cell, or a protoplast of a plant or the like. Therefore, it is not necessary to use a special device such as phase contrast microscope as conventionally used.

Next, as a second embodiment to which the present invention has been applied, an observing method using the cell observing tool as described above will be described for more detail.

The observing method according to the present invention is characterized in that micro transparent object such as a cell can be observed with enhanced contrast. Here, prior to explaining the observing method according to the present invention, a principle will be briefly described, regarding the observation of the micro transparent object with enhanced contrast by the observing method according to the present invention.

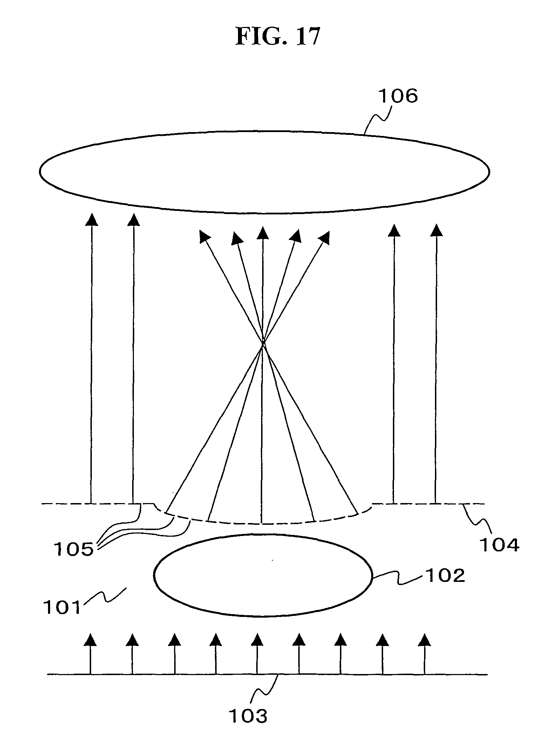

In the conventional transmission observation microscope, FIG. 17 shows how to form a contrast to an object as an observation target. In other words, when an incident wavefront 103 of the illumination light is allowed to pass through the observation target 102 in the medium 101, an injection wavefront 104 injected the observation target 102 is subjected to deformation, since the refractive index of the medium 101 is different from that of the object 102. Minor elements 105 of the injection wavefront proceed in a direction perpendicular to each wavefront. Therefore, the minor elements 105 of the injection wavefront having been deformed proceeds in a traveling direction different from that of the incident wavefront 104. The objective lens 106 forms an object image on a wavefront within a certain angular range indicated by the numerical aperture. When a part of the minor elements 105 of the injection wavefront which has been drastically deformed by periphery of the observation target 102 proceeds out of the angular range indicated by the numerical aperture of the objective lens 106, a shadow occurs in a part of the image, and an image of the objection target 102 is formed with a contrast. However, if there is not a large difference in refractive index between the object 102 and the medium 101, for example in the case of a cell in a culture solution, the micro elements 105 of the injection wavefront having been deformed also proceed within the angular range indicated by the numerical aperture of the object lens 106, and thus, light-dark contrast is hardly given to the object image. Therefore, it has been difficult to observe an image of the cell within the culture solution by use of a general transmission observation microscope.

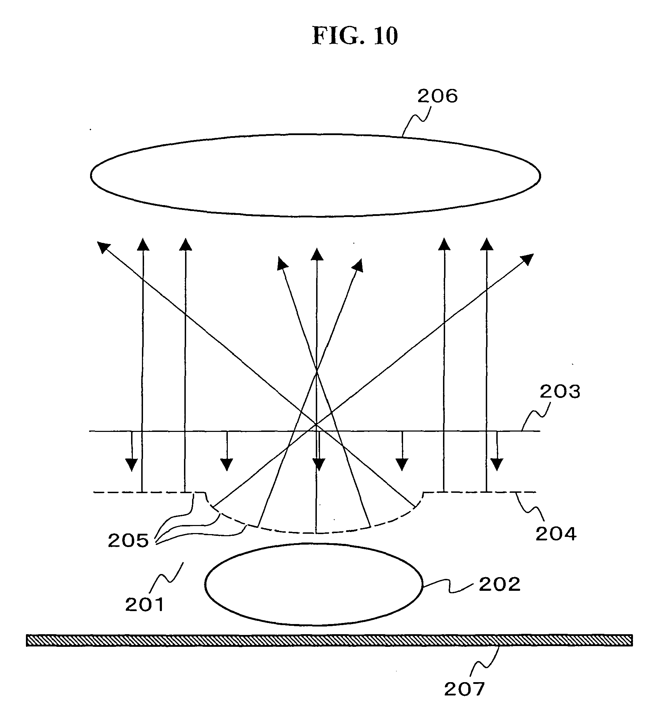

On the other hand, FIG. 10 shows how to form contrast to an object of the observation target in the observing method according to the present invention. In other words, the object 202 in proximity to the reflection plane 207 is observed using the vertical lighting, whereby the incident wavefront 203 is once allowed to pass through the observation target 202, and after it is reflected by the reflection plane 207, it is further allowed to pass through the observation target 202 once again. The injection wavefront 204 formed by two-times transmission of the incident wavefront 203 through the observation target 202 is subjected to deformation twice as much as the case of conventional transmission observation microscope. In the conventional transmission observation microscope, a part of the micro element 205 of the injection wavefront proceeds within the angular range indicated by the numerical aperture of the object lens 206. According to the observing method of the present invention, it proceeds out of the angular range, and a shadow is formed on the image of the observation target. Therefore, a light-dark contrast is provided more easily than the conventional transmission observation microscope. Even the cell in the culture solution, which is hardly observed by the general transmission observation microscope, is allowed to be clearly observed with enhanced contrast.

In the following, the second embodiment of the present invention will be explained with reference to the drawings.

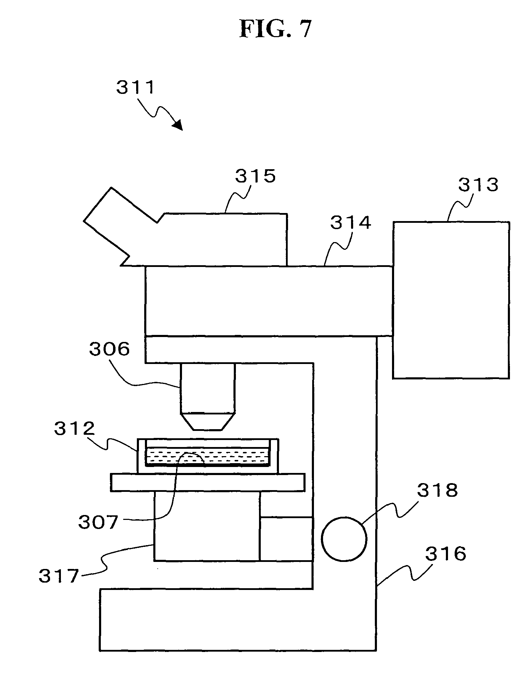

FIG. 7 is a schematic diagram of the observing apparatus to which one embodiment of the present invention has been applied. As is shown, the observing apparatus of the present embodiment has a vertical lighting observation microscope 311, and a culture plate 312 to which a mirror coating 307 is applied onto the bottom thereof.

The vertical lighting observation microscope 311 includes a lens-barrel 315, a stage 317, a light source section 313, a vertical floodlighting tube 314, and an objective lens 306, and a mirror substrate 316 which supports those elements integrally. The stage 317 is designed so that the culture plate 312 is installed on the top surface thereof. The stage 317 is linked with the mirror substrate 316 in such a manner as movable vertically by rotating focusing knob 318.

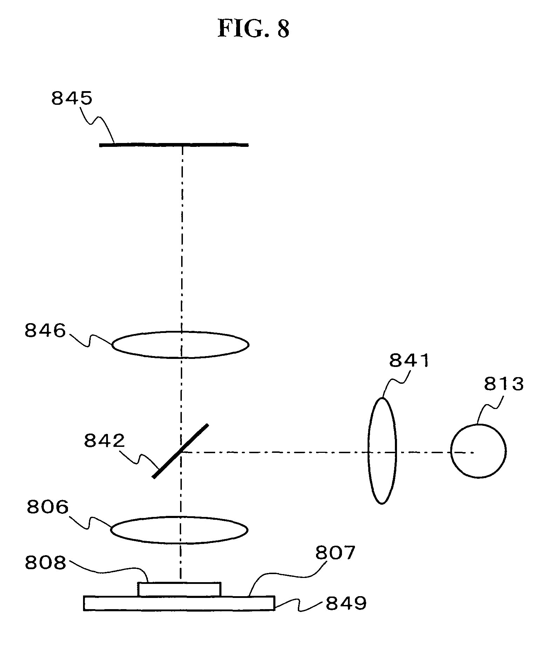

FIG. 8 shows a configuration of optical system of the vertical lighting observation microscope 311. As shown in FIG. 8, this optical system has a configuration including, a light source 813 which generates illumination lights, a collimating lens 841 which allows the illumination lights generated by the light source 813 to proceed to the same traveling direction, a semi-transparent mirror 842 which deflects the traveling direction of the illumination lights proceeding to the same direction into the vertical direction, an objective lens 806 which collects the illumination lights onto a sample 808 including an observation object and extends and projects the sample 808, an imaging lens 846 which forms an image of the sample 808 on an imaging field 845, and a mirror 849 having a reflection plane 807 which reflects and returns the illumination light having once transmitted the sample 808.

Imaging lens 846 is stored in the lens-barrel 315. The light source 813 is stored in the light source section 313, and the collimating lens 841 and the semi-transparent mirror 842 are stored in the vertical floodlighting tube 314.

When the observation target is observed by use of the observing apparatus configured as described above, the following procedure will be taken.

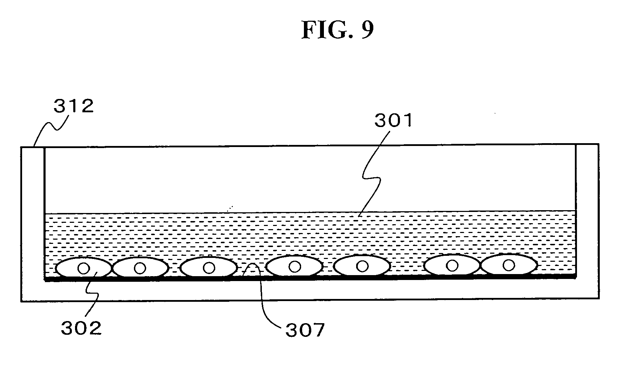

As shown in FIG. 9, the observation target (cell 302, for example) is put in the storing section of the observing tool together with the culture solution 301. The culture plate 312 is installed on the top surface of the stage 317. Brightness of the illumination light from the light source 313 is controlled appropriately. The focusing knob 318 is rotated to move the stage 317 vertically, and observation is performed while taking the focus.

In the observing method according to the present embodiment, it is preferable that the distance $d$ between the observation object and the reflection plane satisfies the formula as described below (1).

$$d \le W/(2NA^2) \quad \ldots \quad (1)$$

(In the formula, $d$ represents a distance between the observation target and the reflection plane, W represents a light wavelength used for observation, and $NA$ represents numerical apertures of the optical system.)

Distance d between the observation target and the reflection plane can be adjusted by controlling the medium density. For example, if the density of the observation target is higher than that of the medium, the observation target automatically precipitates onto the bottom of the culture plate 312 by gravity action, and comes close to the reflection plane on the bottom.

In the following, there will be explained a principle that the observation can be performed with enhanced contrast by satisfying the above formula. When the above formula is satisfied, the observation target is installed in such a manner as coming close to the reflection plane at a distance within around half of the focal depth of the optical system to be observed. Then, the incident wavefront once passed through the observation target passes again the observation target keeping the shape almost as it is. Accordingly, the injection wavefront is clearly bent, in particular, at the edge of the observation target. Therefore, an image of the observation target can be observed with enhanced contrast. In the following, more detailed explanation will be given with a simulation.

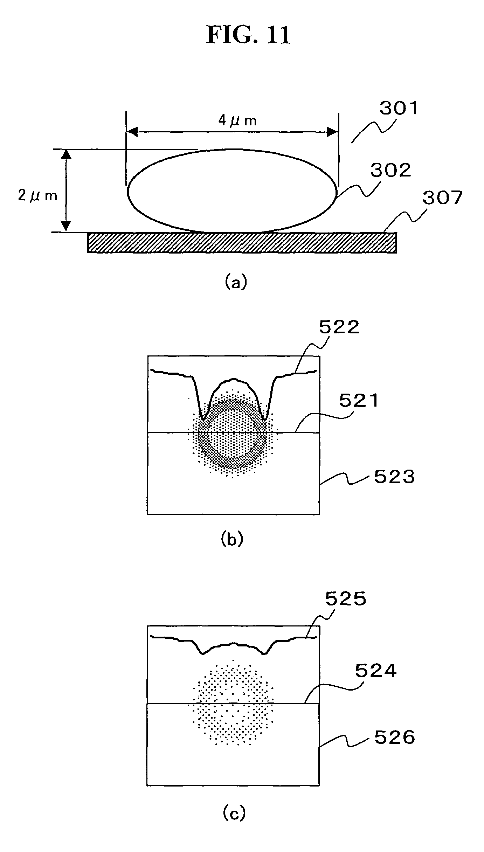

FIG. 11 shows a result of imaging simulation using a calculation model that the cell 302 is assumed as a spheroid having a diameter of $4\mu m$ and a height of $2\mu m$, the refractive index of the cell 302 is 1.4, and the refractive index of the culture solution 301 is 1.33. FIG. 11 is a sketch of image formation. Here, numeral 521 indicates a central line, numeral 522 indicates intensity distribution on the central line, and numeral 523 indicates a formed image of the cell 302. The wavelength of the light used for the observation is assumed as 550 nm. In the simulation in the present embodiment, the vertical lighting passes through the cell 302 two times by reciprocating, whereby the outline of the cell 302 can be observed with a clear light-dark contrast as shown in FIG. 11 (b). On the other hand, as a result of image formation simulation when the cell 302 under the same condition is observed by a conventional transmission observing method, the outline of the cell 302 is not clear as shown in FIG. 11(c).

However, when the cell 302 is more distant from the mirror coating 307, the contrast of the cell image is lowered. The focal depth $\Delta$ of the observation optical system in the present embodiment is obtained by the following formula.

$$\Delta = W/NA^2 = 0.55\mu m/0.452 = 2.7\mu m$$

(In the formula, $W$ represents a wavelength of the light being used, $NA$ represents numerical aperture of the objective lens 306 mounted on the vertical lighting observation microscope 311.)

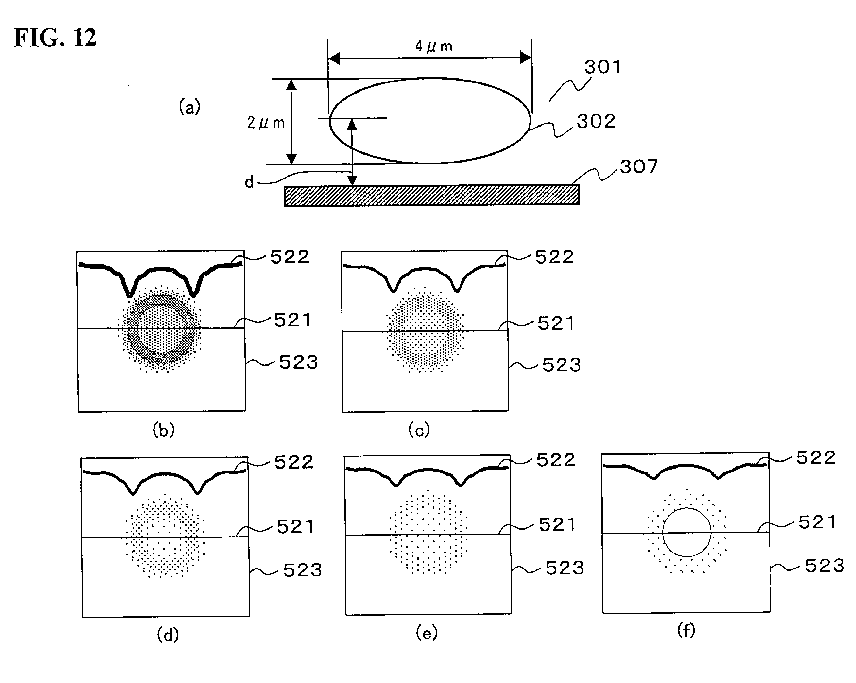

In other words, half of the focal *depth* $\Delta$ is approximately $1.4\mu m$. As shown in FIG. 12(a), image formation simulation is performed by use of the calculation model by changing distance d between the cell 302 and the mirror coating 307, and results of the simulation are shown in FIG. 12(b) to (f). Distance $d$ is $1\mu m$ for (b), $1.2\mu m$ for (c), $1.4\mu m$ for (d), $1.6\mu m$ for (e), and $1.8\mu m$ for (f). As shown in the figures, the contrast of the outline of the cell 302 is lowered as the distance $d$ becomes larger from $1\mu m$, and when the distance $d$ goes beyond $1.4\mu m$, the outline is almost invisible.

Accordingly, it is found to be preferable that the distance between the cell 302 and the mirror coating 307 is around half or less than the focal depth $\Delta$.

In one example of the present embodiment, it is preferable that distance $d$ between the observation target and the reflection plane satisfies the following formula (2).

$$d > F/(4\tan(\sin^{-1} NA)) \quad \ldots \quad (2)$$

(In the formula, $d$ represents a distance between the observation target and the reflection plane, $F$ represents a diameter of vision field of the optical system to observe the observation target, and $NA$ represents a numerical aperture of the optical system to observe the observation target.)

When the observing tool is used as shown in FIG. 3 (A2), the observation target is positioned on the cover glass 5 by gravitation. Therefore, by controlling the depth $a$ of the depressed area of the storage section 2 by a processing, the distance $d$ between the observation target and the reflection plane can be adjusted.

In the case of the observing tool such as the one as shown in FIG. 1 (C), the depth of the depressed area is adjusted by a processing, and a distance between the bottom of the depressed area and the reflection plane is controlled, whereby the distance between the observation target and the reflection plane can be adjusted. In that case of the observing tool such as the one as shown in FIG. 2(B), a thickness of the structure 40 is controlled, whereby the distance between the observation target and the reflection plane can be adjusted.

In addition, the distance $d$ between the observation target and the reflection plane can be adjusted by controlling the medium density. For example, if the density of the observation target is lower than that of the medium, the observation target automatically separates from the bottom of the culture plate 312, and it is positioned at a certain distant from the reflection plane installed on the bottom.

As for the principle to observe with enhanced contrast by satisfying the above formula 2, it will be explained as the following. When the formula 2 is satisfied, the reflection plane goes away for a certain distance from the observation target. Then, the numerical aperture of the illumination light is substantially lowered. Therefore, it is possible to observe the object with enhanced contrast.

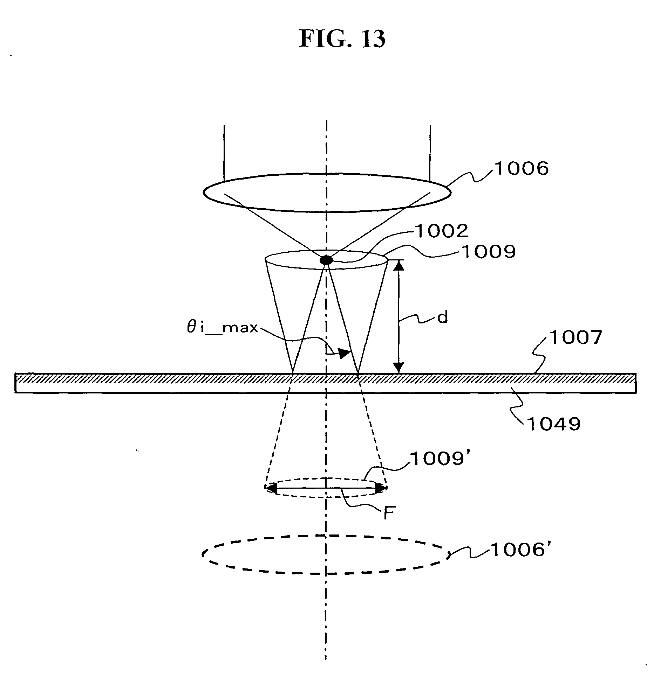

More concretely, as shown in FIG. 13, a situation is considered where an object 1002 at the focal position of the objective lens 1006 is observed using a vertical lighting, with a mirror 1049 having the reflection plane 1007 at a distance d from the object 1002. The radiation field 1009, which directly illuminates the proximity of the object 1002 from the objective lens 1006, is limited by the diameter F, according to the specification of the objective lens 1006 or the observation optical system including the objective lens. The illumination light which is injected from the objective lens 1006, once passes through the proximity of the object 1002, reflected by the reflection plane 1007, and again illuminates the object 1002, appears just like illuminating the object 1002 by use of the mirror image 1009' of the radiation field 1009 serving as a light source plane, with the mirror image function of the reflection plane 1007. In this case, the maximum angle $\theta i\_max$ of the illumination light viewed from the object 1002 is obtained by the following formula.

$$\tan\theta i\_max = F/(4d) \ \ldots \ (3)$$

Accordingly, the numerical aperture $\sin\theta i\_max$ of substantial illumination light against the object 1002 is obtained by the formula;

$$\sin\theta i\_max = \sin(\tan^{-1} F/(4d)) \ \ \ldots \ (4)$$

If the condition is that the above numerical aperture is smaller than the numerical aperture $NA$ of the objective lens 1006;

$$NA > \sin\theta i\_max \ \ldots \ \text{FORMULA 5,}$$

that is,

$$d > F/(4\tan(\sin^{-1}NA)) \ \ldots \ (2),$$

the numerical aperture of the substantial illumination light is smaller than the numerical aperture $NA$ of the objective lens, thereby improving the contrast of the observed image of the object 1002.

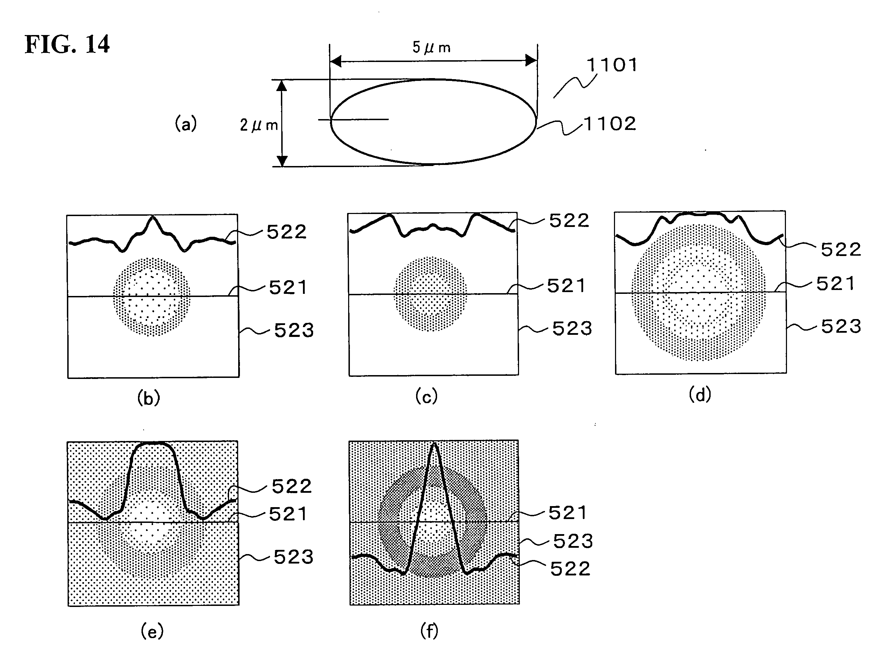

A simulation example will be explained with reference to FIG. 14, where the contrast of the observed image of the object is improved when the numerical aperture of the illumination light is smaller than that of the objective lens. FIG. 14(a) shows a calculation model that a spheroid shaped cell 1102 having a diameter of $5\mu m$ and thickness of $2\mu m$ is placed in the culture solution 1101. Here, it is assumed that refractive index of the cell is assumed as 1.4, and that of the culture solution is assumed as 1.33. The wavelength of the illumination light is assumed as 550 nm. The numerical aperture of the objective lens is assumed as 0.45. FIG. 14 (b) to (f) are showing calculation examples respectively when the numerical aperture of the illumination light is (b) 100%, (c) 80%, (d) 60%, (e) 40%, and (f) 20% of the numerical aperture of the objective lens. It is found that as the numerical aperture of the illumination light becomes smaller than the numerical aperture of the objective lens, the contrast of the object image is improved more.

Next, a micro flow-channel observing apparatus will be explained as a third embodiment to which the present invention has been applied. The micro flow-channel observing apparatus according to the third embodiment features the observation target storage section, and it is suitable for observing cell movement.

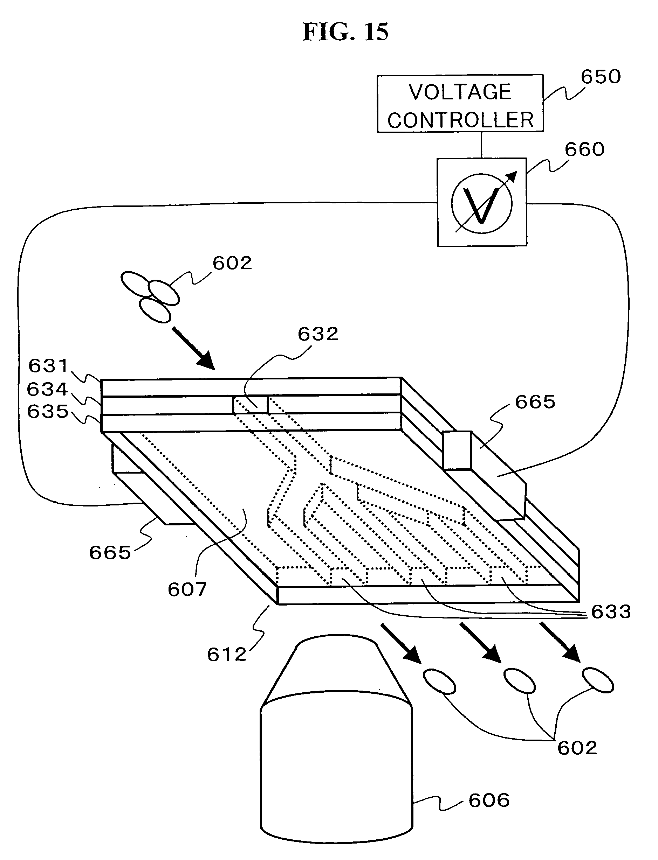

As shown in FIG. 15, the observation target storage section has a micro flow-channel 612. The micro flow-channel 612 is established on a silicon substrate 631. In the present embodiment, the observation target is observed from the lower side by an inverted vertical lighting microscope, not illustrated. In FIG. 15, reference numeral 606 indicates an objective lens.

The micro flow-channel 612 is provided with one inlet 632 and three outlets 633. The inlet 632 and the outlet 633 are connected inside the micro flow-channel 612. The micro flow-channel 612 is manufactured by utilizing a semiconductor manufacturing technique to perform pattern formation with oxide silicon 634 on the silicon substrate, and covering with a glass plate 635 so that the pattern is covered.

The film thickness of the oxide silicon 634 is formed to be almost the same as the thickness of the cell 602. Therefore, when the cell 602 passes through the micro flow-channel 612, the cell 602 substantially comes into contact with the surface 607 of the silicon substrate, whereby the outline of the cell 602 can be observed with enhanced contrast at any time.

It is also possible to configure such that a portion as a cell reservoir is formed adjacent to the inlet 632 of the micro flow-channel 612, and the cell 602 flowing through the micro flow-channel 612 is reserved temporarily. In that case, by making a deep hole on the silicon substrate at the part of the cell reservoir, the surface of the silicon substrate acting as a reflection plane at that part is set to be far away from the glass plate 635. As thus configured, since substantial numerical aperture of the illumination light becomes lower at the part of this cell reservoir, it is possible to observe the cell 602 in the cell reservoir with enhanced contrast.

As shown in the figure, a voltage controller 650, a variable voltage generator 660, and field generator comprising two electrodes 665 are attached to the micro flow-channel 612. The two electrodes 665 are respectively attached to side surfaces of the micro flow-channel 612, and a voltage generated in the variable voltage generator 660 being electrically connected, is applied to the side surfaces of the micro flow-channel 612, thereby generating electric field inside the micro flow-channel. The variable voltage generator 660 is electrically connected to the voltage controller, and based on the method for observing the cell 602 by use of the inverted vertical lighting microscope not illustrated, the voltage generated in the variable voltage generator 660 is controlled so that the cell 602 is allowed to proceed directing to any one of the three outlets 633. A plurality of cells 602 sequentially flow into the micro flow-channel 612 from the inlet 632, and those cells are observed with a vertical lighting via the objective lens 606. The surface 607 of the silicon substrate acts as the reflection plane, whereby the cells 602 inside the micro flow-channel 612 can be observed with enhanced contrast. Based on this information thus observed, the electric field generator changes the electric field intensity within the micro flow-channel 612. The cell 602 within the micro flow-channel 612 is divided into the three outlets 633 to be discharged, the traveling direction thereof being controlled by the internal electric field intensity.

As thus described, by use of the micro flow-channel observing apparatus according to the present embodiment, since a vertical lighting microscope is employed as a means for observing the cell 602, it is possible to establish the micro flowing-channel 612 on the silicon substrate 631. Since a semiconductor manufacturing technique can be applied to the micro flow-channel on the silicon substrate 631, it can be manufactured more inexpensively and in larger quantities, compared to the case where the micro flow-channel is established on a conventional glass substrate. Furthermore, compared to the observing method which employs a conventional special microscope, a transillumination apparatus is not necessary any more, and a stage to hold the micro flow-channel 612 is simplified. Therefore, the entire observing apparatus can be downsized, and it can be established inexpensively.

In addition, also in the third embodiment, it is preferable that a distance between the observation target and the reflection plane (mirror plane) satisfies the formula (1) and formula (2), as explained in the second embodiment.

Embodiments of the present invention have been explained as mentioned above.

In the embodiments above, it is possible to observe a micro transparent object with enhanced contrast, observation of which has been extremely difficult conventionally.

In addition, according to the above embodiments, since an optical system for the transillumination is not necessary, there is an advantage that the entire observing apparatus can be downsized. Furthermore, even when it is difficult to move the object, observation part of the object can be easily adjusted by shifting the entire observing apparatus.

According to the above embodiments, a reflection plane is provided within the container, and thus it is easy to implement a state where the object comes close to the reflection plane. When a reflection plane is provided on the bottom of a petri dish, and medium including the object to be observed is poured into the petri dish, if the density of the object is higher than the medium, the object is automatically precipitated onto the bottom of the petri dish by gravity action, and comes close to the reflection plane on the bottom.

It is to be noted here that the present invention is not limited to the above embodiments, and various modifications are possible within the scope of the invention.

(Example)

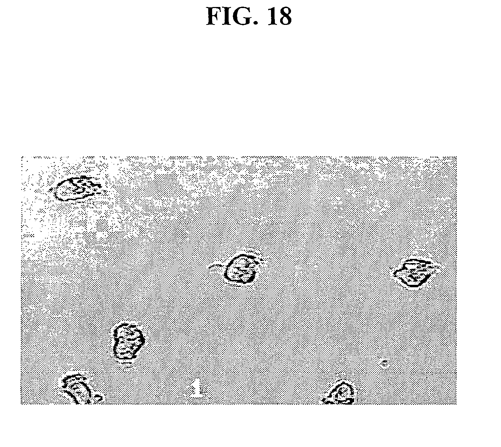

FIG. 18 is a photograph showing the case where a cell is observed and photographed from the lower side, using the observing tool as shown in FIG. 3(A2). Structure 1 is made'of silicon wafer, and an observing tool having a distance *a* being 5μm between the cover glass 5 and the reflection plane, is used. Condition for photographing is as the following;

Photographic apparatus: CCD digital video camera CL-211 H (Watec America Co., Las Vegas, Nev) Lighting system: EPI-U (Nikon, Kawasaki, Japan) Objective lens: ×20

Culture solution: RPMI 1640 buffer solution added with 20mM HEPES and 0.1% bovine serum albumin was used.

Cell: Acidophilic leucocyte

Acidophilic leucocyte refined by a negative selection by the magnetism beads coupled with anti-CD 16 immune body against granulocyte fractionation in human being blood, was used. As for the magnetic beads, Dynal magnetic particle concentrator (Dynal A. S., Oslo, Norway) was used, and an operation was conducted according to a usage method attached to the product.

As shown in FIG. 18, it is found that acidophilic cell and intracellular organelle being micro transparent objects were allowed to be observed with enhanced contrast.

(Description of the Marks)

1 ... STRUCTURE SUCH AS GLASS, STRUCTURE MADE OF PLASTICS, METAL, SILICON WAFER, AND THE LIKE 2 ... CELL STORAGE SECTION, 3 ... SURFACE FORMING A MIRROR, 4 ... MIRROR MADE BY METAL PLATING, OR FOIL OF METAL, SILICON WAFER, AND THE LIKE, 5 ... COVER GLASS, 6 ... CELL INLET, 7, 8 ... HOLE THROUGH WHICH LIQUID PASSES THROUGH, 9 ... CELL STORAGE SECTION FORMED BY TUBE, 10 ... TRANSPARENT INNER SURFACE OF TUBE, 11 ... INNER SURFACE OF TUBE ON WHICH MIRROR IS FORMED, 12 ... TUBE, 15 ... SCALE, 40 ... STRUCTURE SUCH AS GLASS, 41 TO 45 ... SURFACE ON WHICH REFLECTION PLANE IS FORMED, 101, 201 ... MEDIUM, 102, 202, 102 ... OBJECT, 103, 203 ... INCIDENT WAVEFRONT, 104, 204 ... INJECTION WAVEFRONT, 105, 205 ... MICRO ELEMENT ON THE INJECTION WAVEFRONT, 106, 206, 306, 606, 706, 806, 1006 ... OBJECTIVE LENS, 207, 807, 1007 ... REFLECTION PLANE, 301, 1101 ... CULTURE SOLUTION , 302, 602, 1102 ... CELL, 307 ... MIRROR COATING, 311 ... VERTICAL LIGHTING OBSERVATION MICROSCOPE, 312 ... PETRI DISH, 313, 713, 813 ... LIGHT SOURCE, 314 ... VERTICAL FLOODLIGHTING TUBE, 315 ... LENS-BARREL, 316 ... MIRROR SUBSTRATE, 317, 717 ... STAGE, 318 ... FOCUSING KNOB, 521 ... CENTRAL LINE, 522 ... INTENSITY DISTRIBUTION ON THE CENTRAL LINE, 523 ... IMAGE FORMATION OF CELL, 524 ... CENTRAL LINE, 525 ... INTENSITY DISTRIBUTION ON THE CENTRAL LINE, 526 ... IMAGE FORMATION OF CELL, 607 ... SURFACE OF SILICON BASE, 612 ... MICRO FLOW-CHANNEL, 631 ... SILICON BASE, 632 ... INLET, 633 ... OUTLET, 634 ... OXIDE SILICON, 635 ... GLASS PLATE, 708, 808 ... SAMPLE

**Claims**

1. An observing tool comprising an observation target storage section having a mirror.

2. An observing tool used for storing an observation target that is used in an observing method which observes an observation target, by illuminating the target with vertical lighting via an optical system having an objective lens, comprising an-reflection plane which reflects said vertical lighting when the observation is performed.

3. The observing tool according to claim 2, wherein,
   said reflection plane is provided on a surface to be facing to the objective lens when the observation is performed.

4. The observing tool according to claim 2, wherein,
   said reflection plane is provided on a surface opposite to the surface that is to be facing to the objective lens when the observation is performed.

5. The observing tool according to any one of claims 2 to 4, comprising,
   a flow-channel which said observation target passes through.

6. The observing tool according to any one of claims 2 to 5, wherein,
   a storage section to store said observation target comprises an inlet to inject liquid containing the observation target, and an outlet to, flow the liquid out.

7. An observing method which observes an observation target by illuminating the target with vertical lighting via an optical system having an objective lens, wherein,

an observing tool which stores said observation target is provided with a reflection plane to reflect said vertical lighting when observation is performed, and
said observation target is stored in said observing tool and said observation target is observed.

8. The observing method according to claim 7, wherein,
said reflection plane is provided on a surface to be facing to the objective lens when the observation is performed.

9. The observing method according to claim 7, wherein,
said reflection plane is provided on a surface opposite to the surface that is to be facing to the objective lens when the observation is performed.

10. The observing method according to any one of claims 7 to 9,
said observation target is a micro transparent object.

11. The observing method according to any one of claims 7 to 10, wherein,
said observing tool has a container to hold liquid, and
said container stores the observation target with the liquid containing the observation target.

12. The observing method according to claim 11, wherein,
said observation target is a cell, and
said liquid is a culture solution.

13. The observing method according to any one of claims 7 to 12, wherein,
said observation target is stored in said observing tool so that a distance between said observation target and said reflection plane becomes a half or less than the focal depth of said optical system.

14. The observing method according to any one of claims 7 to 13, wherein,
said observation target is stored in said observing tool so that distance $d$ between the observation target and the reflection plane satisfies the following formula (1),

$$d \leq W/(2NA^2) \quad \ldots \quad (1)$$

(in the formula, $d$ represents the distance between the observation target and the reflection plane, $W$ represents a wavelength of the light employed in the observation, and $NA$ represents a numerical aperture of the optical system).

15. The observing method according to any one of claims 7 to 14, wherein,
said observation target is stored in said observing tool so that the numerical aperture of the illumination light against the observation target becomes smaller than the numerical apertures of the objective lens.

16. The observing method according to any one of claims 7 to 15, wherein,
said observation target is stored in said observing tool so that distance d between the observation target and the reflection plane satisfies the following formula (2),

$$d > F/(4\tan(\sin^{-1}NA))$$

(in the formula, $d$ represents the distance between the observation target and the reflection plane, $F$ represents a visual field diameter of the optical system, and $NA$ represents a numerical aperture of the optical system.)

# FIG. 1

(A)

(B)

(C)

# FIG. 2

(A)

(B)

# FIG. 3

(A1)

(A2)

(B)

(C)

# FIG.4

(A)

(B)

(C)

# FIG. 5

# FIG. 6

(A)

(B)

# FIG. 7

**FIG. 8**

FIG. 9

# FIG. 10

# FIG. 11

(a)

(b)

(c)

FIG. 12

## FIG. 13

# FIG. 14

(a)

5 μm

2 μm

1101

1102

(b)

522

521

523

(c)

522

521

523

(d)

522

521

523

(e)

522

521

523

(f)

521

523

522

# FIG. 15

# FIG. 16

745

746

748

706

708

717

744

747

743

742

741

713

# FIG. 17

# FIG. 18

## INTERNATIONAL SEARCH REPORT

| International application No. |
|---|
| PCT/JP2004/004209 |

**A. CLASSIFICATION OF SUBJECT MATTER**
Int.Cl⁷ G02B21/00, C12M1/34

According to International Patent Classification (IPC) or to both national classification and IPC

**B. FIELDS SEARCHED**

Minimum documentation searched (classification system followed by classification symbols)
Int.Cl⁷ G02B21/00, C12M1/34

Documentation searched other than minimum documentation to the extent that such documents are included in the fields searched
Jitsuyo Shinan Koho          1922–1996     Toroku Jitsuyo Shinan Koho    1994–2004
Kokai Jitsuyo Shinan Koho    1971–2004     Jitsuyo Shinan Toroku Koho    1996–2004

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 09-061360 A  (Mitsubishi Cable Industries, Ltd.),<br>07 March, 1997 (07.03.97),<br>Par. Nos. [0023] to [0028], [0031] to [0036];<br>Figs. 1, 2<br>(Family: none) | 1-11,13,14<br>12,15,16 |
| X<br>Y | JP 2001-228404 A  (Nikon Engineering Co., Ltd.),<br>24 August, 2001 (24.08.01),<br>Par. Nos. [0009] to [0011], [0020], [0022];<br>Fig. 1<br>(Family: none) | 1-4,7-10<br>5,6,11-16 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>09 June, 2004 (09.06.04) | Date of mailing of the international search report<br>22 June, 2004 (22.06.04) |
|---|---|
| Name and mailing address of the ISA/<br>Japanese Patent Office | Authorized officer |
| Facsimile No. | Telephone No. |

Form PCT/ISA/210 (second sheet) (January 2004)

<div align="center">

**INTERNATIONAL SEARCH REPORT**

</div>

| | International application No. |
|---|---|
| | PCT/JP2004/004209 |

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 09-051792 A  (Kabushiki Kaisha Hidan),<br>25 February, 1997 (25.02.97),<br>Par. Nos. [0001], [0002], [0004], [0006];<br>Fig. 1<br>(Family: none) | 5,6,11-16 |
| Y | JP 05-027179 A  (Fuji Photo Film Co., Ltd.),<br>05 February, 1993 (05.02.93),<br>Par. Nos. [0004], [0005], [0016] to [0018];<br>Fig. 1<br>(Family: none) | 15,16 |

Form PCT/ISA/210 (continuation of second sheet) (January 2004)